# EUROPÄISCHE PATENTSCHRIFT

(11) **EP 2 797 579 B1**
(45) Veröffentlichungstag und Bekanntmachung des Hinweises auf die Patenterteilung: **29.08.2018**
(21) Anmeldenummer: 12816646.9
(22) Anmeldetag: 19.12.2012
(51) Int. Cl.: A61K 8/97, A61K 39/395

(54) **ZUSAMMENSETZUNG ENTHALTEND ANTIKÖRPER GEGEN GLUTEN UND GERBSTOFFE**
COMPOSITION COMPRISING ANTI-GLUTEN ANTIBODIES AND TANNINS
COMPOSITION COMPRENANT DES ANTICORPS ANTI-GLUTEN ET DES TANINS

(30) Priorität: 19.12.2011 AT 500102011
(43) Veröffentlichungstag der Anmeldung: 05.11.2014
(73) Patentinhaber: STADA Arzneimittel AG, 61118 Bad Vilbel (DE)
(72) Erfinder: MISSBICHLER, Albert, A-1210 Wien (AT)
(74) Vertreter: Sonn & Partner Patentanwälte
(86) Internationale Anmeldenummer: PCT/AT2012/050200
(87) Internationale Veröffentlichungsnummer: WO 2013/090965

(56) Entgegenhaltungen:
- WO-A2-2008/119012
- US-A1- 2011 008 362
- ARRANZ S ET AL: "Analysis of polyphenols in cereals may be improved performing acidic hydrolysis: A study in wheat flour and wheat bran and cereals of the diet", JOURNAL OF CEREAL SCIENCE, ACADEMIC PRESS LTD, XX, Bd. 51, Nr. 3, 1. Mai 2010 (2010-05-01), Seiten 313-318, XP027058788, ISSN: 0733-5210 [gefunden am 2010-02-19]
- Nn: "Tannacomp", Medice , 1. März 2008 (2008-03-01), Seiten 1-2, XP055065878, Gefunden im Internet: URL:http://www.tannacomp.de/servicebereich /downloads/23626_MEDI_EVB_Handzettel.pdf [gefunden am 2013-06-10]

## Beschreibung

Die vorliegende Erfindung betrifft eine Zusammensetzung zur Behandlung von Erkrankungen im Darmtrakt, welche durch Gluten und verwandte Substanzen ausgelöst werden.

Entzündliche Prozesse im Darmbereich können vielfältige Ursachen haben, diese sind in den meisten Fällen schwierig zu diagnostizieren und noch schwieriger zu therapieren. In den letzten Jahren hat die Diagnose der so genannten Lebensmittelunverträglichkeiten einen zunehmend wichtigen Stellenwert in der medizinischen Fachwelt bekommen und damit auch neue Erkenntnisse für die Ursachenerkennung von entzündlichen Prozessen im Darmbereich geliefert.

Viele Menschen entwickeln auch eine Unverträglichkeit gegenüber Histamin und anderen biogenen Aminen. In diesem Fall ist die Leistung des Enzyms Diaminooxidase nicht ausreichend, um die über die Nahrung aufgenommenen biogenen Amine effizient abzubauen. In der Folge gelangt das überschüssige Histamin ins Blut. Typischerweise entwickeln die Betroffenen nach dem Verzehr bestimmter Lebensmittel Kopfschmerzen, Durchfälle, unangenehmen Blähungen und andere allergieähnliche Symptomen.

Sehr komplex stellt sich die Gluten-Unverträglichkeit, auch Zöliakie oder Sprue genannt, dar. Diese Krankheit ist eine vielschichtige Kombination von Resorptionsstörung und interagierender Autoimmunreaktion des Körpers, welche teilweise auch eine genetische HLA-Disposition, nämlich den HLA-DQ2 oder DQ8 Serotyp benötigen. Bislang ist unklar, warum der Großteil der Bevölkerung mit dieser genetischen Disposition einen Toleranzmechanismus entwickelt. Derzeit ist eine Vielzahl von Definitionen von Krankheitsbildern, welche durch Gluten bzw. Gliadin ausgelöst werden, in der Literatur zu finden. Diese umfasst unter anderem:
Asymptomatische Zöliakie (auch Stille Zöliakie): Keine Symptome bei Gluten-Anwesenheit oder Entzug; Antikörper vorhanden; erhöhtes Risiko für Ausbruch einer schweren Zöliakie.
Klassische Zöliakie: Klassische Symptome von Malabsorbtion; Durchfall, Fettstuhl, Gewichtsverlust, Gedeihstörungen. Meist bei Kindern diagnostiziert.
Subklinische Zöliakie: Eher unspezifische Symptome wie Eisenmangel, Leberfunktionsstörungen, ewtl. Biopsieresultate vorhanden.
Symptomatische Zöliakie: Gastrointestinale/extraintestinale Symptome bei Gluten-Einnahme; breites Symptomspektrum.
Refraktäre Zöliakie (RCD): Malabsorbtion & Zottenatrophie nach mehr als 12 Monaten strikter Gluten freier Diät. Serologie meist negativ; positive Serologie weißt auf Diätfehler hin RCD Typ I: Normaler Phänotyp der interepithelialen Lymphocyten RCD Typ II: Ungebremste klonale Expansion von interepithelialen Lymphocyten, Vorstufe zu Enteropathie-assoziiertem Lymphom.
Latente Zöliakie: Positive Serologie ohne histologische Auffälligkeiten des Dünndarms oder normale Histologie der Dünndarmmucosa, die sich auf Glutengabe ändert oder nichtdiagnostizierte Zöliakie oder morphologische Abweichungen der Dünndarmmucosa ohne auffällige Serologie.
Potentielle Zöliakie: Positive Serologie, keine Zottenatrophie.
Celiac Disease Autoimmunity: Positive Serologie, gekennzeichnet durch erhöhte tTG ("tissue transglutaminase"; Gewebetransglutaminase) oder EMA ("Endomysial components of antibodies") Levels an zumindest 2 Messzeitpunken.
Gluten Intoleranz: Synonym für Zöliakie oder eine unspezifische Zöliakie-ähnliche Erkrankung, die auf eine Gluten freie Diät anspricht.
Non-celiac gluten sensitivity (NCGS): Überbegriff immunologischer, morphologischer oder anderer Symptome, die im Zusammenhang mit Gluteneinnahme auftreten (können). Angeborenes Immunsystem ist aktiviert, aber negative Serologie und keine Zottenatrophie. Hier handelt es sich um eine lokale Gluten-Allergie mit nur lokaler IgE Ausschüttung.
Gluten-assoziierten Störungen: Gluten Ataxie, Morbus Duhring (Dermatitis Herpetiformis).

Biochemisch - physiologisch völlig anders gelagert ist die Gluten-Allergie ("Mehl-Allergie"). Dabei handelt es sich um eine klassische IgE-vermittelte Allergie gegen Gluten, wobei die Sensibilisierung der Lunge meist durch Mehlstaub erfolgt.

Die nicht allergische Immunreaktion der Zöliakie wird hauptsächlich durch die alkohollöslichen Proteinfraktionen (so genannte Prolamine) von Weizen (*Gliadin*)*,* Roggen (*Secalin*), Gerste (*Hordein*) und Hafer (*Avenin*) und deren Bruchstücke nach dem peptisch-tryptischen Verdau im Magen ausgelöst.

Die Gesamtheit dieser Proteine wird im Folgenden als Gluten zugehörige Proteine ("gluten related proteins") bezeichnet. Diese Proteine induzieren in vitro und in vivo reproduzierbar das immunologische Reaktionsspektrum der Zöliakie. Sie enthalten charakteristischerweise "Aminosäurewiederholungen", die aus Prolinen und Glutaminen bestehen. Beispielhaft seien die typischen Peptide erwähnt: α-Gliadin: LQLQPF(PQPLPY)₃PQPQPF; γ-Gliadin: FLQPQQPF(PQQ)₂PY(PQQ)₂PFPQ; LMW-Glutenin: QQQQPPFSQQQQSPFSQQQQ; HMW-Glutenin: (GYYPTSPQQ)ₙ. Grundsätzlicher Auslöser des gesamten physiologischen Prozesses der Zöliakie ist die Passage des im Magen anverdauten Glutens durch die epitheliale Zellschicht des Dünndarms in die dahinter liegende Lamina.

Die US 2011/0008362 A1 betrifft die Verwendung von Antikörpern gerichtet gegen Gluten zur Behandlung und Prävention von Erkrankungen, die im Zusammenhang mit Zöliakie stehen. Diese Antikörper werden in Hühnereiern hergestellt, wobei der Eidotter, der diese Antikörper umfasst, vor der Verabreichung zu einem Lebensmittel verarbeitet werden kann. Gemäß Fig. 1 der US 2011/0008362 A1 soll der Antikörper umfassende Eidotter mit Weizenmehl vermischt und anschließend zu einem anti-Gluten-Antikörper-enthaltenden Brot weiterverarbeitet werden können.

Aufgabe der vorliegenden Erfindung ist es Mittel zur Verfügung zu stellen, welche die entzündlichen oder immunologischen Prozesse, welche durch eine oder mehrere der Gluten zugehörigen Proteine ("gluten related proteins") im Darmtrakt ausgelöst werden zu verhindern, zu reduzieren oder zu lindern.

Daher betrifft die vorliegende Erfindung eine Zusammensetzung zur Verwendung zur Behandlung einer Erkrankung des Darmtrakts, welche durch ein Gluten zugehöriges Protein ausgelöst wird, umfassend mindestens ein Mittel, welches an das Gluten zugehörige Protein bindet, dadurch gekennzeichnet, dass die Zusammensetzung gleichzeitig oder maximal innerhalb von 60 (vorzugsweise 50, noch mehr bevorzugt 40, noch mehr bevorzugt 30) Minuten nach oraler Verabreichung mindestens eines Gerbstoffs in einer Menge von 100 bis 10000 mg einem Patienten oral verabreicht wird, wobei das mindestens eine Mittel, welches an das Gluten zugehörige Protein bindet, ein Antikörper, ein Antikörperfragment, ein Aptamer und/oder ein DARpin ("Designed Ankyrin Repeat Proteins") oder ein spezifischer Rezeptor gerichtet gegen das Gluten zugehörige Protein ist. Erfindungsgemäß wird die Aufgabe durch die Kombination zweier Mittel gelöst, die in zeitlicher Abfolge oral zugeführt werden. Die vorliegende Erfindung stellt erstmals eine funktionelle Kombination von zwei grundlegend unterschiedlichen Wirkungsweisen dar. Zunächst wird die Darmmucosa mit Hilfe von Gerbstoffen stabilisiert und die Resorption potentiell toxischer Substanzen stark reduziert. Gleichzeitig oder unmittelbar anschließend werden die toxischen Substanzen und/oder Mediatoren vermittels spezifischer Adduktoren gebunden und damit dem physiologischen Aufnahmeprozess entzogen.

Eine Aufgabe, nämlich die Bindung oder Maskierung der Gluten zugehörigen Proteine ("gluten related proteins"), wird erfindungsgemäß durch spezifische Antikörper, Antikörperfragmente (z.B. leichte Ketten, Antigen-spezifische Bindungsstellen und dgl.), Aptamere, DARPIN's oder andere geeignete spezifische Bindeproteinstrukturen, die dem Fachmann bekannt sind, erfüllt. Die Herstellung dieser maskierenden Substanzen kann dem jeweiligen Stand der Technik angepasst und optimiert werden.

Bevorzugt sind Antikörper oder deren Fragmente, welche Legehennen im Anschluss an eine Immunisierung mit einem gewünschten Antigen in den Eidotter sezernieren. Diese Immunglobuline (IgY) ähneln in ihrer Struktur den Säuger-Immunglobulinen IgG und IgE, interagieren jedoch auf Grund des Fehlens der FC-Domäne nicht mit dem Säuger-Immunsystem.

Da die Antikörper in natürlicher, bioaktiver Form in den Darm eingebracht werden müssen, bieten sich bevorzugt IgY aus Eidotter an, da hier eine Interaktion mit FC-Rezeptoren von Immunzellen, Rheumatoid Factor, sowie dem Komplementsystem ausgeschlossen werden kann. Dieser Ansatz von spezifischen IgY gegen Gluten wurde bereits durchgeführt (siehe z.B. US 2011/0008362). Alternativ zu den IgY können naturgemäß auch andere, polyklonale oder monoklonale Antikörper oder deren Fragmente eingesetzt werden, wenn sie durch Methoden, welche dem Stand der Technik entsprechen modifiziert und humanverträglich gemacht wurden. Weitere Antikörper im Sinne der Erfindung können aus Echsen oder dem Blut von Lungenfischen gewonnen werden. Weiters bevorzugt im Sinne der Erfindung ist der Einsatz von Aptameren, welche speziell jene Epitope erkennen, welche die unerwünschten Reaktionen im Magen-Darm-Bereich auslösen oder steuern. Die Entwicklung und Charakterisierung dieser Aptamere ist dem Fachmann bekannt.

Der Begriff "Gluten zugehörige Proteine" ("gluten related proteins"), wie hierin verwendet, umfasst Gliadin, Secalin, Hordein und Avenin und deren Bruchstücke nach dem peptisch-tryptischen Verdau im Magen, wobei besonders bevorzugt Gliadin und dessen Bruchstücke sind. Daher kann als Synonym für "Gluten zugehörige Proteine" "Gliadin, Secalin, Hordein und Avenin und deren Bruchstücke nach dem peptisch-tryptischen Verdau im Magen", "Gliadin, Secalin, Hordein und Avenin und durch Pepsin und/oder Trypsin daraus hergestellte Bruchstücke (Fragmente)" oder "Gliadin, Secalin, Hordein, Avenin und Fragmente davon" verwendet werden. Fragmente im Sinne der vorliegenden Erfindung bestehen aus 5 bis 100, vorzugsweise 10 bis 50, Aminosäuren.

Die Gerbstoffe erfüllen die Aufgabe der Stabilisierung der Darmmucosa. Gerbstoffe stellen eine weite Familie von verwandten, polyphenolischen Substanzen dar, welche in höheren Pflanzen und Algen vorkommen. Sie sind im Bausteinprinzip verzweigte Makromoleküle, die gewisse physikalisch-chemische Eigenschaften teilen: Wasserlöslichkeit, und die Fähigkeit, sich an Proteine anzulagern und diese zu vernetzen, Bildung von Niederschlägen mit Alkaloiden sowie die Bindung von Chelatkomplexen mit Fe^{III}-Salzen. Erfindungsgemäß können unterschiedliche Gerbstoffe, auch in gemischter Form, für die Stabilisierung der Mucosa eingesetzt werden. Bevorzugt werden Gerbstoffe der Typen Catechingerbstoffe, Tannine, Lamiaceen-Gerbstoffe oder Algen-Gerbstoffe eingesetzt. Quellen für Gerbstoffen sind z.B: Galläpfel (Gallae); Hamamelisblätter (Hamamelidis folium); Walnussblätter (Juglandis folium; Eichenrinde (Quercus cortex); Ratanhiawurzel (Ratanhiae radix); Blutwurzwurzel (Tormentillae rhizoma); Heidelbeeren (Myrtilli fructus); Catechu; Rubus fruticosus; Potentilla anserina; Fragaria vesca; Agrimonia eupatoria; Alchemilla xanthochlora; Plantago major; Plantago lanceolata, Rosa gallica; Sanguisorba officinalis oder auch Kerne von Datteln, Trauben usw. Fakultativ können erfindungsgemäß auch synthetische Gerbstoffe wie z.B. Polymere von Acrylaten, Polyurethanen, Isocyanaten, Derivate von Aldehyden aber auch Salze von Mineralstoffen wie z.B: Alaune, Chromsalze oder Zirkonsalze eingesetzt werden.

Gemäß einer besonders bevorzugten Ausführungsform ist der mindestens eine Gerbstoff ausgewählt aus der Gruppe bestehend aus Catechingerbstoffe, Tannine, Lamiaceen-Gerbstoffe und Algen-Gerbstoffe.

Die Fähigkeit, Proteine zu vernetzen beruht auf einer unspezifischen Anlagerung der phenolischen Strukturen an hydrophobe Taschen, sowie der Ausbildung von Wasserstoffbrücken zwischen den Hydroxylgruppen des Gerbstoffes und den Seitenketten polarer Aminosäuren. Die Anlagerung der Gerbstoffe führt zu einer Auffaltung und bei einer ausreichenden Konzentration zur Quervernetzung der Proteine.

Besonders bevorzugt werden die Gerbstoffe eingesetzt, nachdem sie mit Protein im Verhältnis 20:80 [Gerbstoff:Protein]; bevorzugt 35:65, besonders bevorzugt 50:50 vorgefällt (z.B. durch Inkontaktbringen des Gerbstoffs mit dem Protein/Polypeptid) werden. Als Protein können generell Proteine mit einer Molekularmasse zwischen 50 kDa und 500 kDa eingesetzt werden

Überaschenderweise kann durch die Vorfällung des Gerbstoffes mit einem Antikörper oder Bindeprotein ein doppelter Effekt erzielt werden: das entstehende Makromolekül vereinigt die Fähigkeit der Vernetzung der Darmmucosa mit der gleichzeitigen Fähigkeit, toxische Antigene und Substanzen zu binden und deren Bioverfügbarkeit zu reduzieren. Ganz besonders bevorzugt ist daher der Einsatz von Bindeproteinstrukturen, welche mit einem Gerbstoff im Verhältnis 20:80 [Gerbstoff:Protein]; bevorzugt 35:65, besonders bevorzugt 50:50 vorgefällt werden.

Daher umfasst die erfindungsgemäße Zusammensetzung zur Anwendung vorzugsweise ferner mindestens einen Gerbstoff mit dem Ziel der Behandlung von entzündlichen oder nicht allergischen immunologischen Prozessen, welche durch eine oder mehrere der Gluten zugehörigen Proteine ("gluten related proteins") im Darmtrakt ausgelöst werden.

Zusätzlich können neben der Substanz zur Bindung oder Maskierung der Gluten zugehörigen Proteine ("gluten related proteins") und dem Gerbstoff auch bioaktive Mikroorganismen verabreicht werden, die den Abbau dieser toxischen Substanzen und/oder Mediatoren unterstützen und somit potentielle weitere pathologische Prozesse unterbinden.

In einer weiteren Ausformung der erfindungsgemäßen Zusammensetzung zur Anwendung können die spezifisch gebundenen toxischen Substanzen, Peptide bzw. Antigene oder Mediatoren, welche mit dem Speisebrei im Darm weiterbewegt wurden, in weiterer Folge durch die Gabe von speziellen bakteriellen Proteasen völlig abgebaut werden. Im Falle von Zöliakie ist das Enzym der Wahl die in allen Lebewesen vorhandene Prolylendopeptidase (PEP; EC 3.4.21.26). Sie kann Peptide, welche aus Gluten und Gliadin entstehen, als einziges Enzym einem bestimmten Ausmass abauen. PEP kommt primär als intrazelluläres Enzym vor, einige Microorganismen, z.B. Flavobacterium meningosepticum, A. niger, sowie diverse Lactobacilli, sind imstande, dieses Enzym auch zu sezernieren. Weitere für die Erfindung geeignete Mikroorganismen sind unter anderem Bakterien aus dem menschlichen Mundraum wie z.B. Fusobakterien oder Actinomyceten.

Die Verabreichung der Bindeproteinstrukturen und Gerbstoffe erfolgt vorzugsweise in einem definierten zeitlichen Abstand: zunächst wird in einem Zeitraum von 0 bis 60 min, bevorzugt 10 bis 40 min, besonders bevorzugt 15 bis 30 min vor Einnahme der glutenhaltigen Nahrung das Gerbstoffpräparat oral verabreicht

Gemäß einer bevorzugten Ausführungsform der vorliegenden Erfindung ist der Antikörper, Antikörperfragment, Aptamer und/oder DARpin gegen Gliadin und/oder Fragmenten davon, insbesondere gegen tryptisch und/oder peptisch gespaltenes Gliadin, oder physiologisch äquivalente Verdaue von Gluten oder Gluten-Fraktionen gerichtet.

Der mindestens eine Antikörper ist vorzugsweise rekombinanten Ursprungs.

Gemäß einer noch weiteren bevorzugten Ausführungsform der vorliegenden Erfindung ist der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen.

Gemäß einer bevorzugten Ausführungsform der vorliegenden Erfindung umfasst die erfindungsgemäße Zusammensetzung zur Anwendung eukaryotische und/oder prokaryotische Zellen, die in der Lage sind entzündliche Prozesse im Magen und/oder Darmtrakts zu reduzieren und/oder zu unterbinden, wobei die eukaryotischen und/oder prokaryotischen Zellen aus-gewählt sind aus der Gruppe bestehend aus Flavobacterium sp, Lactobacillus sp, Aspergillus sp und Bifidobacterium sp.

Diese Zellen sind vorzugsweise in der Lage Proteine (wie zum Beispiel Enzyme) zu sekretieren, die in der Lage sind entzündliche Prozesse im Darmtrakt zu reduzieren bzw. zu unterbinden, die für die Bildung einer entzündlichen Erkrankung verantwortlich sind.

Gemäß einer besonders bevorzugten Ausführungsform wird die Zusammensetzung umfassend mindestens einen Gerbstoff zur Behandlung einer entzündlichen Erkrankung des Darmtrakts, in Kombination mit mindestens einem zweiten Mittel zur Behandlung einer entzündlichen Erkrankung des Darmtrakts verabreicht, wobei das mindestens eine zweite Mittel mindestens ein Protein oder Polypeptid, welches mindestens eine Substanz (aktiv) bindet, welches die entzündliche Erkrankung induziert oder mediiert, und eukaryotische und/oder prokaryotische Zellen umfasst, die in der Lage sind entzündliche Prozesse im Magen und/oder Darmtrakts zu reduzieren und/oder zu unterbinden. D.h. die Zusammensetzung umfassend mindestens einen Gerbstoff wird in Kombination mit einer Zusammensetzung umfassend zumindest ein Protein oder Polypeptid und mit einer Zusammensetzung umfassend eukaryotische und/oder prokaryotische Zellen, beides wie oben definiert, verabreicht. Die beiden zuletzt genannten Zusammensetzungen können entweder in einer oder mindestens zwei einzelnen Darreichungsformen zur Verabreichung bereitgestellt werden.

Gemäß einer bevorzugten Ausführungsform der vorliegenden Erfindung sind die eukaryotischen und/oder prokaryotischen Zellen ausgewählt aus der Gruppe bestehend aus Flavobacterium sp, Lactobacillus sp, Aspergillus sp und Bifidobacterium sp.

Der mindestens eine Gerbstoff ist vorzugsweise mit mindestens einem Protein vorgefällt.

Gemäß einer weiteren bevorzugten Ausführungsform der vorliegenden Erfindung liegt das mindestens eine Mittel, welches an das Gluten zugehörige Protein bindet, und der mindestens eine Gerbstoff in einer Formulierung zur kontrollierten Freisetzung im Magen und/oder Darmtrakt, vorzugsweise Darmtrakt, vor.

Dem Fachmann sind Mittel zum Formulieren von Wirkstoffen bekannt, die es ermöglichen eine Zusammensetzung bereitzustellen, welche die Eigenschaft aufweist unter bestimmten Bedingungen die in der Zusammensetzung eingearbeiteten Wirkstoffe freizusetzen. Pharmazeutisch werden derartige Mittel dazu eingesetzt und Wirkstoffe zum Beispiel ausschließlich Darmtrakt freizusetzen. Dies hat den Vorteil, dass die Wirkstoffe magensaftresistent durch den Magen in den Darm geschleust werden können.

Der Begriff "Formulierung zur kontrollierten Freisetzung" umfasst nicht nur magensaftresistente Darreichungsformen bzw. Formulierungen sondern auch "Delayed release" zur Freisetzung im Darm mit Zeitverzögerung, Zuckerabbaubare Polymerüberzüge zur Freisetzung im Dickdarm, Überzug als Gelbarriere zur zielgerichteten Freisetzung im Darm je nach Dicke der Barriere, Wasserunlösliche Überzüge mit Porenbildner retardiert die Freisetzung je nach Porengrüße und Menge, pH-sensitive Überzüge zur Freisetzung entsprechend dem physiologischen pH-Gradienten, bioadhärente Überzüge (Chitosan, Polyacrylate) interagieren mit dem Mucos und halten die Arzneiform fest, flotierende Arzneiformen zur Freisetzung im Magen dadurch, dass im Magen Quellung eintritt und der Durchtritt durch den Pylorus verhindert wird, Verarbeitung in Pellets wenn kleiner als 1 bis 3 mm für einen raschen Durchtritt der Wirkstoffe durch den Pylorus und Transfer in den Darm.

Es hat sich erfindungsgemäß als besonders vorteilhaft erwiesen, dass die Gerbstoffe vor der Zusammensetzung umfassend das mindestens eine Mittel, welches an das Gluten zugehörige Protein bindet, verabreicht wird, da dadurch die Benetzung der Schleimhäute, insbesondere Darmschleimhäute, mit den erfindungsgemäß eingesetzten Gerbstoffen vor dem Eintreffen der zuvor genannten Mittel erfolgt. Dadurch wird es ermöglicht, zunächst die Schleimhäute zu stabilisieren, woraufhin die eigentlichen Wirkstoffe zur Behandlung der entzündlichen Erkrankung verabreicht werden.

Die Zusammensetzung umfassend mindestens einen Gerbstoff wird daher mindestens eine, vorzugsweise mindestens fünf, noch mehr bevorzugt mindestens zehn, am meisten bevorzugt mindestens 20, Minuten vor dem mindestens einem Mittel zur Behandlung einer entzündlichen Erkrankung des Darmtrakts verabreicht.

Gemäß einer bevorzugten Ausführungsform der vorliegenden Erfindung wird der mindestens eine Gerbstoff in einer Menge von 10 bis 10000 mg, vorzugsweise von 20 bis 5000 mg, noch mehr bevorzugt von 50 bis 2500 mg, besonders bevorzugt von 100 bis 2000 mg, verabreicht.

Gemäß einer weiteren bevorzugten Ausführungsform der vorliegenden Erfindung wird das mindestens eine Protein oder Polypeptid, welches an mindestens eine Substanz (z.B. Antigen) bindet, welche die Gluten-Unverträglichkeit induziert oder mediiert, in einer Menge von 1 bis 100000 mg, vorzugsweise von 5 bis 50.000 mg, noch mehr bevorzugt von 10 bis 20.000 mg, besonders bevorzugt von 15 bis 15.000 mg, verabreicht.

Gemäß einer besonders bevorzugten Ausführungsform der vorliegenden Erfindung, werden die eukaryotischen und/oder prokaryotischen Zellen in einer Menge von 10⁹ bis 10¹⁴, vorzugsweise von 10¹⁰ bis 10¹³, verabreicht.

Die Verabreichung der erfindungsgemäß eingesetzten Bindeproteinstrukturen und Gerbstoffe erfolgt erfindungsgemäß in einem definierten zeitlichen Abstand: zunächst wird in einem Zeitraum von 0 - 60 min, bevorzugt 10 bis 40 min, besonders bevorzugt 15 bis 30 min vor Einnahme der glutenhaltigen Nahrung das Gerbstoffpräparat oral verabreicht

Bei oraler Verabreichung der erfindungsgemäßen Zusammensetzungen werden diese entsprechend formuliert (zum Beispiel magensaftresistent) .

Ein noch weiterer Aspekt der vorliegenden Erfindung betrifft ein Set zur Verwendung zur Behandlung einer Erkrankung des Darmtrakts, welche durch ein Gluten zugehöriges Protein ausgelöst wird, umfassend mindestens ein Behältnis mit mindestens einen Gerbstoff, wobei der Gerbstoff oral verabreicht wird, und mindestens ein weiteres Behältnis mit einer Zusammensetzung, wobei die Zusammensetzung mindestens ein Mittel, welches an das Gluten zugehörige Protein bindet, umfasst, wobei das mindestens eine Mittel, welches an das Gluten zugehörige Protein bindet, ein Antikörper, ein Antikörperfragment, ein Aptamer und/oder ein DARpin ("Designed Ankyrin Repeat Proteins") oder ein spezifischer Rezeptor gerichtet gegen das Gluten zugehörige Protein ist, wobei die Zusammensetzung oral verabreicht wird.

Gemäß einer bevorzugten Ausführungsform der vorliegenden Erfindung umfasst das Set Zusammensetzungen wie oben definiert und wird zu den oben angeführten Zwecken eingesetzt und der oben beschriebenen Art verabreicht.

## Patentansprüche

1. Zusammensetzung zur Anwendung in der Behandlung einer Erkrankung des Darmtrakts, welche durch ein Gluten zugehöriges Protein ausgelöst wird, umfassend mindestens ein Mittel, welches an das Gluten zugehörige Protein bindet, **dadurch gekennzeichnet, dass** die Zusammensetzung gleichzeitig oder maximal innerhalb von 60 Minuten nach oraler Verabreichung mindestens eines Gerbstoffs in einer Menge von 100 bis 10000 mg einem Patienten oral verabreicht wird,
wobei das mindestens eine Mittel, welches an das Gluten zugehörige Protein bindet, ein Antikörper, ein Antikörperfragment, ein Aptamer und/oder ein DARpin (*"Designed Ankyrin Repeat Proteins")* oder ein spezifischer Rezeptor gerichtet gegen das Gluten zugehörige Protein ist.

2. Zusammensetzung zur Anwendung gemäß Anspruch 1, **dadurch gekennzeichnet, dass** die Erkrankung des Darmtrakts, welche durch eine oder mehrere Gluten zugehörigen Proteine ausgelöst wird Zöliakie ist.

3. Zusammensetzung zur Anwendung gemäß Anspruch 2, **dadurch gekennzeichnet, dass** die Zöliakie ausgewählt ist aus der Gruppe bestehend aus asymptomatischer Zöliakie, klassischer Zöliakie, subklinischer Zöliakie, symptomatischer Zöliakie, refraktärer Zöliakie, latenter zöliakie, potentieller Zöliakie, Celiac Disease Autoimmunity, Gluten-Intoleranz, Non-celiac gluten sensitivity und Gluten-assoziierten Störungen.

4. Zusammensetzung zur Anwendung gemäß einem der Ansprüche 1 bis 3, **dadurch gekennzeichnet, dass** das Gluten zugehöriges Protein ausgewählt ist aus der Gruppe bestehend aus Gliadin, Secalin, Hordein, Avenin und Fragmente davon.

5. Zusammensetzung zur Anwendung gemäß einem der Ansprüche 1 bis 4, **dadurch gekennzeichnet, dass** das Gluten zugehörige Protein Gliadin oder ein Fragment davon ist.

6. Zusammensetzung zur Anwendung gemäß einem der Ansprüche 1 bis 5, **dadurch gekennzeichnet, dass** der mindestens eine Antikörper ein polyklonaler Antikörper aus Säugetieren, polyklonaler Antikörper avianen Ursprungs, ein Antikörper aus Echsen, ein monoklonaler Antikörper oder ein Antikörper aus dem Blut von Lungenfischen ist.

7. Zusammensetzung zur Anwendung gemäß einem der Ansprüche 1 bis 6, **dadurch gekennzeichnet, dass** die Zusammensetzung eukaryotische und/oder prokaryotische Zellen umfasst, die in der Lage sind, entzündliche Prozesse im Magen und/oder Darmtrakts zu reduzieren und/oder zu unterbinden; wobei die eukaryotischen und/oder prokaryotischen Zellen ausgewählt sind aus der Gruppe bestehend aus Flavobacterium sp, Lactobacillus sp, Aspergillus sp und Bifidobacterium sp.

8. Zusammensetzung zur Anwendung gemäß einem der Ansprüche 1 bis 7, **dadurch gekennzeichnet, dass** der mindestens eine Gerbstoff ausgewählt ist aus der Gruppe bestehend aus Catechingerbstoffe, Tannine, Lamiaceen-Gerbstoffe und Algen-Gerbstoffe.

9. Zusammensetzung zur Anwendung gemäß einem der Ansprüche 1 bis 8, **dadurch gekennzeichnet, dass** der mindestens eine Gerbstoff mit mindestens einem Protein vorgefällt ist.

10. Zusammensetzung zur Anwendung gemäß einem der Ansprüche 1 bis 9, **dadurch gekennzeichnet, dass**:
das mindestens eine Mittel, welches an das Gluten zugehörige Protein bindet, und der mindestens eine Gerbstoff in einer Formulierung zur kontrollierten Freisetzung im Magen und/oder Darmtrakt, vorzugsweise Darmtrakt, vorliegt; und/oder der mindestens eine Gerbstoff mindestens eine, vorzugsweise mindestens fünf, noch mehr bevorzugt mindestens zehn, am meisten bevorzugt mindestens 20, Minuten vor der Zusammensetzung umfassend das mindestens eine Mittel, welches an das Gluten zugehörige Protein bindet, verabreicht wird; und/oder
der mindestens eine Gerbstoff in einer Menge von 100 bis 5000 mg, noch mehr bevorzugt von 100 bis 2500 mg, besonders bevorzugt von 500 bis 2000 mg, verabreicht wird; und/oder
das Mittel, welches an das Gluten zugehörige Protein bindet, in einer Menge von 200 bis 100000 mg, vorzugsweise von 200 bis 50.000 mg, noch mehr bevorzugt von 200 bis 20.000 mg, besonders bevorzugt von 500 bis 15.000 mg, verabreicht wird.

11. Zusammensetzung zur Anwendung gemäß einem der Ansprüche 7 bis 10, **dadurch gekennzeichnet, dass** die eukaryotischen und/oder prokaryotischen Zellen in einer Menge von 10⁹ bis 10¹⁴, vorzugsweise von 10¹⁰ bis 10¹³, verabreicht werden.

12. Set zur Anwendung in der Behandlung einer Erkrankung des Darmtrakts, welche durch ein Gluten zugehöriges Protein ausgelöst wird, umfassend mindestens ein Behältnis mit mindestens einem Gerbstoff, wobei der Gerbstoff oral verabreicht wird, und mindestens ein weiteres Behältnis mit einer Zusammensetzung, wobei die Zusammensetzung mindestens ein Mittel, welches an das Gluten zugehörige Protein bindet, umfasst, wobei das mindestens eine Mittel, welches an das Gluten zugehörige Protein bindet, ein Antikörper, ein Antikörperfragment, ein Aptamer und/oder ein DARpin (*"Designed Ankyrin Repeat Proteins"*) oder ein spezifischer Rezeptor gerichtet gegen das Gluten zugehörige Protein ist, wobei die Zusammensetzung oral verabreicht wird.

## Claims

1. Composition for use in the treatment of a disease of the intestinal tract, which disease is caused by a gluten-related protein, the composition comprising at least one means that binds to the gluten-related protein, **characterised in that** the composition is orally administered to a patient in an amount of 100 to 10,000 mg at the same time as oral administration of at least one tannin or at most within 60 minutes thereafter, the at least one means that binds to the gluten-related protein being an antibody, an antibody fragment, an aptamer and/or a DARPin (designed ankyrin repeat protein) or a specific receptor directed against the gluten-related protein.

2. Composition for use according to claim 1, **characterised in that** the disease of the intestinal tract, which is caused by a gluten-related protein, is coeliac disease.

3. Composition for use according to claim 2, **characterised in that** the coeliac disease is selected from the group consisting of asymptomatic coeliac disease, classic coeliac disease, subclinical coeliac disease, symptomatic coeliac disease, refractory coeliac disease, latent coeliac disease, potential coeliac disease, coeliac disease autoimmunity, gluten intolerance, non-coeliac gluten sensitivity, and gluten-associated disorders.

4. Composition for use according to any of claims 1 to 3, **characterised in that** the gluten-related protein is selected from the group consisting of gliadin, secalin, hordein, avenin and fragments thereof.

5. Composition for use according to any of claims 1 to 4, **characterised in that** the gluten-related protein is gliadin or a fragment thereof.

6. Composition for use according to any of claims 1 to 5, **characterised in that** the at least one antibody is a polyclonal antibody from mammals, a polyclonal antibody of avian origin, an antibody from lizards, a monoclonal antibody, or an antibody from the blood of lungfish.

7. Composition for use according to any of claims 1 to 6, **characterised in that** the composition comprises eukaryotic and/or prokaryotic cells that are able to reduce and/or prevent inflammatory processes in the stomach and/or intestinal tract, the eukaryotic and/or prokaryotic cells being selected from the group consisting of Flavobacterium sp., Lactobacillus sp., Aspergillus sp. and Bifidobacterium sp.

8. Composition for use according to any of claims 1 to 7, **characterised in that** the at least one tannin is selected from the group consisting of catechin tannins, tannoids, Lamiaceae tannins and algal tannins.

9. Composition for use according to any of claims 1 to 8, **characterised in that** the at least one tannin is precipitated in advance with at least one protein.

10. Composition for use according to any of claims 1 to 9, **characterised in that**:
the at least one means that binds to the gluten-related protein and the at least one tannin formulated for controlled release in the stomach and/or intestinal tract, preferably intestinal tract; and/or
the at least one tannin is administered at least one, preferably at least five, more preferably at least ten, most preferably at least twenty, minutes before the composition comprising the at least one means that binds to the gluten-related protein; and/or
the at least one tannin is administered in an amount of from 100 to 5,000 mg, more preferably of from 100 to 2,500 mg, particularly preferably of from 500 to 2,000 mg; and/or
the means that binds to the gluten-related protein is administered in an amount of from 200 to 100,000 mg, preferably of from 200 to 50,000 mg, more preferably of from 200 to 20,000 mg, particularly preferably of from 500 to 15,000 mg.

11. Composition for use according to any of claims 7 to 10, **characterised in that** the eukaryotic and/or prokaryotic cells are administered in an amount of from 10⁹ to 10¹⁴, preferably of from 10¹⁰ to 10¹³.

12. Set for use in the treatment of a disease of the intestinal tract, which disease is caused by a gluten-related protein, the set comprising at least one container containing at least one tannin, the tannin being administered orally, and at least one additional container containing a composition, the composition comprising at least one means that binds to the gluten-related protein, the at least one means that binds to the gluten-related protein being an antibody, an antibody fragment, an aptamer and/or a DARPin (designed ankyrin repeat protein) or a specific receptor directed against the gluten-related protein, the composition being administered orally.

## Revendications

1. Composition destinée à être utilisée dans le traitement d'une maladie du tractus intestinal, qui est déclenchée par une protéine associée au gluten, comprenant au moins un agent qui se lie à la protéine associée au gluten, **caractérisée en ce que**, la composition est administrée à un patient par voie orale simultanément ou au maximum dans les 60 minutes suivant l'administration orale d'au moins un tanin en une quantité de 100 à 10 000 mg, dans laquelle ledit au moins un agent qui se lie à la protéine associée au gluten est un anticorps, un fragment d'anticorps, un aptamère et/ou une DARpin (« *Designed Ankyrin Repeat Proteins* ») ou un récepteur spécifique dirigé contre la protéine associée au gluten.

2. Composition destinée à être utilisée selon la revendication 1, **caractérisée en ce que** la maladie du tractus intestinal qui est déclenchée par une ou plusieurs protéines associées au gluten est la maladie coeliaque.

3. Composition destinée à être utilisée selon la revendication 2, **caractérisée en ce que** la maladie coeliaque est choisie dans le groupe constitué de la maladie coeliaque asymptomatique, la maladie coeliaque classique, la maladie coeliaque subclinique, la maladie coeliaque symptomatique, la maladie coeliaque réfractaire, la maladie coeliaque latente, la maladie coeliaque potentielle, la maladie coeliaque auto-immune, l'intolérance au gluten, la sensibilité au gluten non coeliaque et les troubles associés au gluten.

4. Composition destinée à être utilisée selon l'une des revendications 1 à 3, **caractérisée en ce que** la protéine associée au gluten est choisie dans le groupe constitué de la gliadine, la sécaline, la hordéine, l'avénine, et des fragments de celles-ci.

5. Composition destinée à être utilisée selon l'une des revendications 1 à 4, **caractérisée en ce que** la protéine associée au gluten est la gliadine ou un fragment de celle-ci.

6. Composition destinée à être utilisée selon l'une des revendications 1 à 5, **caractérisée en ce que** ledit au moins un anticorps est un anticorps polyclonal provenant de mammifères, un anticorps polyclonal d'origine aviaire, un anticorps de lézard, un anticorps monoclonal ou un anticorps provenant du sang de dipneustes.

7. Composition destinée à être utilisée selon l'une des revendications 1 à 6, **caractérisée en ce que** la composition comprend des cellules eucaryotes et/ou procaryotes, qui sont en mesure de réduire et/ou de réprimer les processus inflammatoires dans l'estomac et/ou dans le tractus intestinal ; dans laquelle les cellules eucaryotes et/ou procaryotes sont choisies dans le groupe constitué de Flavobacterium sp., Lactobacillus sp., Aspergillus sp. et Bifidobacterium sp.

8. Composition destinée à être utilisée selon l'une des revendications 1 à 7, **caractérisée en ce que** ledit au moins un tanin est choisi dans le groupe des tanins de catéchine, des tannines, des tanins de lamiacées et des tanins d'algues.

9. Composition destinée à être utilisée selon l'une des revendications 1 à 8, **caractérisée en ce que** ledit au moins un tanin est pré-précipité avec au moins une protéine.

10. Composition destinée à être utilisée selon l'une des revendications 1 à 9, **caractérisée en ce que** :
ledit au moins un agent qui se lie à la protéine associée au gluten, et ledit au moins un tanin se présentent dans une formulation pour la libération contrôlée dans l'estomac et/ou le tractus intestinal, de préférence le tractus intestinal ; et/ou
ledit au moins un tanin est administré au moins une, de préférence au moins cinq, de manière plus préférée au moins dix, de manière la mieux préférée au moins 20, minutes avant la composition comprenant ledit au moins un agent qui se lie à la protéine associée au gluten ; et/ou
ledit au moins un tanin est administré en une quantité de 100 à 5 000 mg, de manière encore plus préférée de 100 à 2 500 mg, de manière particulièrement préférée de 500 à 2 000 mg ; et/ou
l'agent qui se lie à la protéine associée au gluten est administré en une quantité de 200 à 100 000 mg, de préférence 200 à 50 000 mg, de manière encore plus préférée de 200 à 20 000 mg, de manière particulièrement préférée de 500 à 15 000 mg.

11. Composition destinée à être utilisée selon l'une des revendications 7 à 10, **caractérisée en ce que** les cellules eucaryotes et/ou procaryotes sont administrées en une quantité de 10⁹ à 10¹⁴, de préférence 10¹⁰ à 10¹³.

12. Set destiné à être utilisé dans le traitement d'une maladie du tractus intestinal qui est déclenchée par une protéine associée au gluten, comprenant au moins un récipient ayant au moins un tanin, dans lequel le tanin est administré par voie orale, et au moins un autre récipient ayant une composition, dans lequel la composition comprend au moins un agent qui se lie à la protéine liée au gluten, dans lequel ledit au moins un agent qui se lie à la protéine associée au gluten est un anticorps, un fragment d'anticorps, un aptamère et/ou une DARpin (« *Designed Ankyrin Repeat Proteins* ») ou un récepteur spécifique dirigé contre la protéine associée au gluten, dans lequel la composition est administrée par voie orale.
